(19) Europäisches Patentamt / European Patent Office / Office européen des brevets

(11) EP 1 523 464 B1

(12) **EUROPÄISCHE PATENTSCHRIFT**

(45) Veröffentlichungstag und Bekanntmachung des Hinweises auf die Patenterteilung:
**16.01.2008 Patentblatt 2008/03**

(21) Anmeldenummer: **03763837.6**

(22) Anmeldetag: **14.07.2003**

(51) Int Cl.:
***C07C 29/141*** *(2006.01)* ***C07C 33/025*** *(2006.01)*

(86) Internationale Anmeldenummer:
**PCT/EP2003/007599**

(87) Internationale Veröffentlichungsnummer:
**WO 2004/007411 (22.01.2004 Gazette 2004/04)**

(54) **VERFAHREN ZUR KONTINUIERLICHEN HYDRIERUNG VON CITRONELLAL ZU CITRONELLOL**

METHOD FOR CONTINUOUS HYDROGENATION OF CITRONELLAL TO FORM CITRONELLOL

PROCEDE D'HYDROGENATION CONTINUE DE CITRONELLAL EN CITRONELLOL

(84) Benannte Vertragsstaaten:
**AT BE BG CH CY CZ DE DK EE ES FI FR GB GR HU IE IT LI LU MC NL PT RO SE SI SK TR**

(30) Priorität: **15.07.2002 DE 10231942**

(43) Veröffentlichungstag der Anmeldung:
**20.04.2005 Patentblatt 2005/16**

(73) Patentinhaber: **BASF Aktiengesellschaft**
**67056 Ludwigshafen (DE)**

(72) Erfinder:
- **GÖBBEL, Hans-Georg**
  **67169 Kallstadt (DE)**
- **GERLACH, Till**
  **67071 Ludwigshafen (DE)**
- **WEGNER, Günter**
  **67354 Römerberg (DE)**
- **FUCHS, Hartwig**
  **67063 Ludwigshafen (DE)**
- **UNVERRICHT, Signe**
  **68169 Mannheim (DE)**
- **SALDEN, Axel**
  **70180 Stuttgart (DE)**

(74) Vertreter: **Reitstötter - Kinzebach**
**Patentanwälte**
**Sternwartstrasse 4**
**81679 München (DE)**

(56) Entgegenhaltungen:
**EP-A- 1 318 129**
**US-A- 4 029 709**
**US-A- 5 939 589**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 523 464 B1

## Beschreibung

Die vorliegende Erfindung betrifft ein Verfahren zur kontinuierlichen selektiven Hydrierung von Citronellal zu Citronellol (Schema 1).

Schema 1

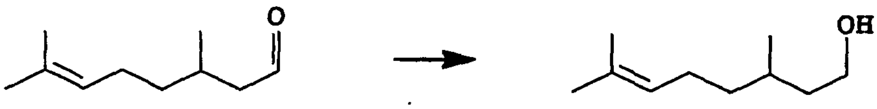

Citronellal Citronellol

Citronellol findet als Riech- und Aromastoff Anwendung.

Die US 3,346,650 offenbart ein Verfahren Zur Herstellung von Citronellol durch Hydrierung eines Gemisches von Geraniol und Nerol an einem Kupferchromatkatalysator.

US,4,029,709 offenbart ein Verfahren zur Herstellung von Citronellal durch Hydrierung von Citronellal an einem Ni/Cr Katalysator.

Katalytische Hydrierungen an heterogenen Katalysatoren werden vielfach unter Einsatz von Festbettreaktoren durchgeführt, um die Vorzüge einer kontinuierlichen Verfahrensführung zu erhalten. Allerdings müssen dafür speziell präparierte Katalysatoren hergestellt und eingesetzt werden, die bei Verlust der Aktivität - oft bereits nach kurzeren Standzeiten - in aufwändiger Weise ausgetauscht oder regeneriert werden müssen, was in der Regel nicht nur mit der Abstellung der Hydrieranlage, sondern auch der nachfolgenden Aufarbeitungsstufen verbunden ist.

Alternativ kann eine heterogen katalysierte Hydrierung in Form einer Suspensionsreaktion durchgeführt werden, wobei der Hydrierkatalysator durch Zufuhr mechanischer Energie z.B. in einem Rührkessel in einer Flüssigphase suspendiert wird, vgl. z.B. Ullmanns Encyklopädie der technischen Chemie, 4. Aufl. Band 13, 1997, S. 138, Verlag Chemie Weinheim. Eine Erhöhung der Energiezufuhr über den zur Suspendierung erforderlichen Betrag führt zu keiner nennenswerten Verbesserung des Stofftransports der zu hydrierenden Moleküle an die Oberfläche der Katalysatorteilchen, da die erzielbare Relativgeschwindigkeit zwischen Katalysatorteilchen und Flüssigphase die Sedimentationsgeschwindigkeit nur unwesentlich übersteigt. Fließ- oder Wirbelbettreaktoren gestatten zwar höhere Relativgeschwindigkeiten, erfordern aber die Verwendung deutlich größerer Katalysatorteilchen, damit im Betrieb ein mehr oder weniger stark expandiertes Katalysatorbett vorliegt. Die geringere volumenbezogene Oberfläche größerer Katalysatorteilchen limitiert aber den Stoffumsatz und kompensiert so den Effekt der höheren Relativgeschwindigkeit.

Die EP-A 798 039 offenbart ein Verfahren zur Durchführung von katalytischen Reaktionen in einem Reaktor, der eine Flüssigphase enthält, in der mindestens ein Katalysator suspendiert ist. Beschrieben wird die Hydrierung von Hydrodehydrolinalool zu Hydrolinalool und weiter zu Tetrahydrolinalool. Hydrodehydrolinalool enthält lediglich eine Dreifachbindung als zu hydrierende funktionelle Gruppe, so dass der Fachmann dieser Schrift keine Anregung bezüglich einer selektiven Hydrierung entnommen hätte.

Der vorliegenden Erfindung liegt die Aufgabe zugrunde, ein Verfahren zur selektiven Hydrierung von Citronellal zu Citronellol anzugeben, das die Vorteile einer hohen Raum-Zeit-Ausbeute und eines einfachen Katalysatoraustausches vereint.

Die Aufgabe wird erfindungsgemäß gelöst durch ein Verfahren zur selektiven Hydrierung von Citronellal zu Citronellol, bei dem man eine Flüssigphase, in der das Citronellal gelöst ist und in der Teilchen eines Katalysators suspendiert sind, der zur präferentiellen Hydrierung von Kohlenstoff-Sauerstoff-Doppelbindungen vor Kohlenstoff-Kohlenstoff-Doppelbindungen fähig ist, in Gegenwart eines wasserstoffhaltigen Gases durch eine Vorrichtung führt, welche den Transport der Katalysatorteilchen hemmt, wobei die den Transport der Katalysatorteilchen hemmende Vorrichtung Öffnungen oder Kanälen aufweist, deren hydraulischer Durchmesser das 2- bis 2000-fache des mittleren Durchmessers der Katalysatorteilchen beträgt, und die Flüssigphase außerdem Ammoniak, ein primäres, sekundäres und/oder tertiäres Amin sowie ein inertes Verdünnungsmittel umfasst und die Konzentration von Citronellal in der Flüssigphase 50 bis 90 Gew.-% beträgt.

Beim erfindungsgemäßen Verfahren wird eine höhere Relativgeschwindigkeit der Flüssigphase gegenüber den Katalysatorteilchen erzeugt, weil der Transport der Katalysatorteilchen durch geeignete Mittel, wie Einbauten in einem Reaktor, gehemmt wird, d.h. die Partikel werden gegenüber der umgebenden Flüssigkeit stärker zurückgehalten. In Verbindung mit der hohen volumenbezogenen Oberfläche der suspendierten Partikel werden im Ergebnis hohe Raum-Zeit-Ausbeuten erzielt.

Eine geeignete Vorrichtung zur Durchführung des erfindungsgemäßen Verfahrens ist in der EP-A 798 039

beschrieben.

Die den Transport der Katalysatorteilchen hemmende Vorrichtung weist Öffnungen oder Kanälen auf, deren hydraulischer Durchmesser das 2- bis 2000-fache, insbesondere das 5- bis 500-fache, besonders bevorzugt das 5- bis 100-fache des mittleren Durchmessers der Katalysatorteilchen beträgt.

Der hydraulische Durchmesser ist eine dem Fachmann geläufige Kenngröße zur Beschreibung des Äquivalentdurchmessers nichtkreisrunder Kanalstrukturen. Der hydraulische Durchmesser einer Öffnung ist als Quotient des 4-fachen Querschnitts der Öffnung und deren Umfang definiert. Bei Kanälen mit einem Querschnitt in Gestalt eines gleichschenkeligen Dreiecks lässt sich der hydraulische Durchmesser als

$$\frac{2bh}{b + 2s}$$

beschreiben, worin b für die Basis, h für die Höhe und s für die Schenkellänge des Dreiecks steht.

Die Öffnungen oder Kanäle geeignete Vorrichtungen weisen im Allgemeinen einen hydraulischen Durchmesser von 0,5 bis 20 mm, vorzugsweise 1 bis 10 mm, besonders bevorzugt 1 bis 3 mm, auf.

Üblicherweise verwendet man Katalysatorteilchen eines mittleren Durchmessers von 0,0001 bis 2 mm, bevorzugt von 0,001 bis 1 mm, besonders bevorzugt von 0,005 bis 0,1 mm.

Die den Transport der Katalysatorteilchen hemmende Vorrichtung kann aus einer Schüttung, einem Gestrick, einer offenzelligen Schaumstruktur, vorzugsweise aus Kunststoff z.B. Polyurethan oder Melaminharz, oder Keramik, oder einem Packungselement, wie es grundsätzlich, d.h. seiner geometrischen Form nach, bereits aus der Destillations- und Extraktionstechnik bekannt ist, bestehen. Für Zwecke der vorliegenden Erfindung haben die Packungen jedoch grundsätzlich einen wesentlich, regelmäßig um den Faktor 2 bis 10 kleineren hydraulischen Durchmesser als vergleichbare Einbauten im Bereich der Destillations- und Extraktionstechnik.

Als Packungselemente eignen sich insbesondere Metallgewebepackungen bzw. Drahtgewebepackungen, z.B. der Bauart Montz A3, Sulzer BX, DX und EX. Anstelle von Metallgewebepackungen können auch Packungen aus anderen gewebten, gewirkten oder gefilzten Materialien verwendet werden. Weiterhin eignen sich Packungen ebener oder gewellter Bleche, bevorzugt ohne Perforation oder andere größere Öffnungen, beispielsweise entsprechend den Bauarten Montz B1 oder Sulzer Mellapak. Vorteilhaft sind auch Packungen aus Streckmetall, wie z.B. Packungen des Typs Montz BSH. Entscheidend für die Eignung einer Packung im Rahmen der vorliegenden Erfindung ist nicht deren Geometrie, sondern die für die Stromführung entstehenden Öffnungsgrößen bzw. Kanalbreiten in der Packung.

In einer bevorzugten Ausführungsform weisen die der Flüssigphase zugewandten Oberflächen der Vorrichtung eine Rauhigkeit im Bereich des 0,1 bis 10-fachen, vorzugsweise des 0,5- bis 5-fachen, des mittleren Durchmessers der Katalysatorteilchen auf. Bevorzugt sind Materialien, deren Oberflächen einen Mittelrauwert $R_a$ (bestimmt nach DIN 4768/1) von 0,001 bis 0,01 mm aufweisen. Eine entsprechende Oberflächenrauhigkeit kann bei Verwendung von Drahtgewebepackungen aus Edelstahl durch thermische Behandlung in Gegenwart von Sauerstoff erreicht werden, z.B. indem man das Gewebe an der Luft bei einer Temperatur von etwa 800 °C tempert.

Das erfindungsgemäße Verfahren erfolgt im Allgemeinen bei einem Druck zwischen 1 und 100 bar, bevorzugt 1 und 60 bar, besonders bevorzugt 1 und 50 bar. Die Reaktionstemperaturen liegen üblicherweise zwischen 40 und 120°C, bevorzugt zwischen 60 und 100°C, besonders bevorzugt zwischen 70 und 90°C.

Erfindungsgemäß umfasst die Flüssigphase neben Citronellal ein inertes Verdünnungsmittel, insbesondere ein $C_1$-$C_6$-Alkanol, besonders bevorzugt ein $C_1$-$C_4$-Alkanol, wie insbesondere Methanol. Weiter umfasst die Flüssigphase außerdem Ammoniak, ein primäres, sekundäres und/oder tertiäres Amin, wovon tertiäre Amine, z.B. Tri($C_1$-$C_4$-alkyl) amine, insbesondere Trimethylamin, besonders bevorzugt sind. Die Konzentration an Citronellal in der Flüssigphase beträgt 50 bis 90 Gew.-%, besonders bevorzugt 60 bis 80 Gew.-%, die des Verdünnungsmittels 40 bis 5 Gew.-%, bevorzugt 20 bis 35 %, die des Ammoniaks/Amins 1 bis 15 Gew.-%, bevorzugt 1 bis 8 Gew.-%.

Als wasserstoffhaltiges Gas verwendet man in der Regel Wasserstoffgas mit einer Reinheit von wenigstens 99,5 Vol.-%. Es wird in wenigstens stöchiometrischer Menge, bezogen auf die in der Flüssigphase enthaltene Carbonylverbindung eingesetzt, meist in einem Überschuß von 1 bis 20 %.

Als Katalysator kann ein handelsüblicher Suspensionskatalysator verwendet werden, der zur präferentiellen Hydrierung von Kohlenstoff-Sauerstoff-Doppelbindungen vor Kohlenstoff-Kohlenstoff-Doppelbindungen fähig ist. Es eignen sich besonders solche Katalysatoren, die als Aktivkomponente mindestens Ruthenium enthalten. Neben Ruthenium kann der Katalysator auch weitere Aktivkomponenten, wie beispielsweise Eisen, enthalten. Der Katalysator kann in metallischer und/oder oxidischer Form eingesetzt werden. Vorzugsweise sind die Aktivkomponenten auf einem Trägermaterial aufgebracht. Als Trägermaterialien eignen sich beispielsweise $SiO_2$, $TiO_2$, $ZrO_2$, $Al_2O_3$ oder Kohlenstoff wie Graphite, Ruße oder Aktivkohle. Aktivkohle ist aufgrund ihrer leichten Suspendierbarkeit bevorzugt. Der Gehalt an

Ruthenium beträgt vorzugsweise 0,1 und 10 Gew.-%, der Gehalt an Eisen vorzugsweise 0,1 und 5 Gew.-%, insbesondere 0,5 und 1,5 Gew.-%, bezogen auf das Gesamtgewicht des Katalysators.

Das suspendierte Katalysatormaterial kann mit Hilfe gebräuchlicher Techniken in die Flüssigphase eingebracht und darin verteilt werden.

Bei der den Transport der Katalysatorteilchen hemmenden Vorrichtung handelt es sich üblicherweise um Einbauten in einem Reaktor, die so angeordnet sind, dass das Reaktionsgemisch beim Passieren des Reaktors durch die Vorrichtung gezwängt wird, d.h. die Einbauten füllen in der Regel den gesamten freien Querschnitt des Reaktors. Die Einbauten erstrecken sich vorzugsweise, aber nicht notwendigerweise über die gesamte Ausdehnung des Reaktors in Strömungsrichtung der Flüssigphase.

Es eignen sich verschiedene Reaktorformen, wie Strahldüsenreaktoren, Blasensäulen oder Rohrbündelreaktoren. Davon sind eine vertikal angeordnete Blasensäule oder ein Rohrbündelreaktor, bei dem die Einbauten in den einzelnen Rohren untergebracht sind, besonders geeignet.

Das wasserstoffhaltige Gas und die Flüssigphase werden bevorzugt im Gleichstrom, vorzugsweise entgegen der Richtung der Schwerkraft, durch den Reaktor geführt. Die Gasphase wird beispielsweise mittels einer Injektordüse innig mit der Flüssigphase durchmischt. Die Leerrohrgeschwindigkeit der Flüssigphase betragen vorzugsweise mehr als 100 $m^3/m^2h$, insbesondere 100 bis 250 $m^3/m^2h$, die der Gasphase vorzugsweise mehr als 100 $Nm^3/m^2h$, insbesondere 100 bis 250 $Nm^3/m^2h$. Um ausreichend hohe Leerrohrgeschwindigkeiten zu erzielen, ist es bevorzugt, Teilströme der Gas- und Flüssigphase, die den Reaktor verlassen, zurückzuführen.

Die im Hydrieraustrag suspendierten Katalysatorteilchen werden durch übliche Verfahren abgetrennt, z.B. durch Sedimentation, Zentrifugation, Kuchenfiltration oder Querstromfiltration.

Die erfindungsgemäße Hydrierung kann sowohl kontinuierlich als auch diskontinuierlich erfolgen, bevorzugt verläuft sie jedoch kontinuierlich.

Das erfindungsgemäße Verfahren wird durch die beigefügte Figur und das nachstehende Beispiel näher veranschaulicht.

Figur 1 zeigt schematisch eine zur Durchführung des erfindungsgemäßen Verfahrens geeignete Anlage mit einem Reaktor (Blasensäule) 1 mit einer Packung 2, die den Transport der Katalysatorteilchen hemmt. In den Rektor 1 werden über die Leitungen 3 Flüssigkeit und über die Leitung 4 Wasserstoffgas eingeführt wird. Das Kreisgas 5 wird mittels der Mischdüse 6 mit Frischgas und der durch die Pumpe 14 im Kreis geführten Suspension 11 eingemischt. Der Reaktoraustrag wird über die Leitung 7 in das Abscheidegefäß 8 gefahren, in dem die Gasphase abgeschieden und über Leitung 9 abgeführt wird. Von dieser Gasmenge wird zur Begrenzung der Aufpegelung von gasförmigen Verunreinigungen ein Teilstrom über die Leitung 10 entnommen und die verbleibende Restmenge über die Leitung 5 in den Reaktor geführt. Der suspendierte Katalysator verbleibt im Reaktorsystem, indem er über einen Querstromfilter 12 zurückgehalten und nur katalysatorfreie Flüssigphase über die Leitung 13 austritt und entnommen wird. Über den Wärmetauscher 15 kann die Temperatur im Reaktorsystem gezielt eingestellt werden.

Figur 2 zeigt schematisch eine Lage eines gefalzten Gewebes. Erfindungsgemäß verwendbare Packungen werden erhalten, wenn mehrere dieser Lagen übereinander angeordnet werden. Jede Lage umfasst Kanäle mit einem Querschnitt in Gestalt eines gleichschenkeligen Dreiecks mit der Schenkellänge s, der Basis b und der Höhe h.

Beispiel 1

Man verwendete eine Anlage wie in Fig. 1 dargestellt, die eine mit einer Gewebepackung vom Typ Montz A1 1200 bestückte Blasensäule (3000 mm Länge, 27,3 mm Durchmesser) umfasste. Die Packung bestand aus übereinander angeordneten Lagen eines Gewebe von Edelstahldrähten, das so gefalzt war, dass Kanäle mit einem Querschnitt in Gestalt eines gleichschenkeligen Dreiecks gebildet werden, wobei die Schenkellänge 3,1 mm, die Basis 5,1 mm und die Höhe 1,8 mm betrug, entsprechend einem hydraulischen Durchmesser von 1,62 mm.

Als Zulauf diente ein Gemisch von 70 Gew.-% Citronellal, 27 Gew.-% Methanol und 3 Gew.-% Trimethylamin. In dem Zulauf wurde ein Ru/Fe-Kohle-Suspensionskatalysator suspendiert, der 5 % Ruthenium und 1 % Eisen auf Aktivkohle enthielt und eine mittlere Korngröße von etwa 50 $\mu$m aufwies. Die Reaktion erfolgte kontinuierlich unter einem Wasserstoffdruck von 20 bar und einer Temperatur von 80°C. Die Flüssigkeit mit dem suspendierten Katalysator und das Gas wurden mit einer Leerrohrgeschwindigkeit von 200 $m^3/m^2h$ von unten in den gepackten Reaktor eingebracht.

Der Umsatz betrug mehr als 95 % bei einer Selektivität von 96 % für Citronellol. Die Katalysatorbelastung betrug 40,2 $kg_{Citronellal}/kg_{Rh}.h$, die Raum-Zeit-Ausbeute 233 $kg_{Citronellol}/m^3.h$.

## Patentansprüche

1. Verfahren zur selektiven Hydrierung von Citronellal zu Citronellol, bei dem man eine Flüssigphase, in der das Citronellal gelöst ist und in der Teilchen eines Katalysators suspendiert sind, der zur präferentiellen Hydrierung von

Kohlenstoff-Sauerstoff-Doppelbindungen vor Kohlenstoff-Kohlenstoff-Doppelbindungen fähig ist, in Gegenwart eines wasserstoffhaltigen Gases durch eine Vorrichtung führt, welche den Transport der Katalysatorteilchen hemmt, wobei die den Transport der Katalysatorteilchen hemmende Vorrichtung Öffnungen oder Kanälen aufweist, deren hydraulischer Durchmesser das 2- bis 2000-fache des mittleren Durchmessers der Katalysatorteilchen beträgt, und die Flüssigphase außerdem Ammoniak, ein primäres, sekundäres und/oder tertiäres Amin sowie ein inertes Verdünnungsmittel umfasst und die Konzentration von Citronellal in der Flüssigphase 50 bis 90 Gew.-% beträgt.

2. Verfahren nach Anspruch 1, wobei die Aktivkomponente des Katalysators Ruthenium enthält.

3. Verfahren nach Anspruch 1 oder 2, wobei man Katalysatorteilchen eines mittleren Durchmessers von 0,0001 bis 2 mm verwendet.

4. Verfahren nach einem der vorhergehenden Ansprüche, wobei man als die den Transport der Katalysatorteilchen hemmende Vorrichtung eine Schüttung, ein Gestrick, eine offenzellige Schaumstruktur oder ein Packungselement verwendet.

5. Verfahren nach einem der vorhergehenden Ansprüche, wobei man die Flüssigphase und das wasserstoffhaltige Gas mit einer Leerrohrgeschwindigkeit von mehr als 100 $m^3/m^2h$ durch die den Transport der Katalysatorteilchen hemmende Vorrichtung führt.

6. Verfahren nach einem der vorhergehenden Ansprüche, wobei die der Flüssigphase zugewandten Oberflächen der Vorrichtung eine Rauhigkeit im Bereich des 0,1 bis 10-fachen des mittleren Durchmessers der Katalysatorteilchen aufweisen.

7. Verfahren nach einem der vorhergehenden Ansprüche, wobei es sich bei dem Verdünnungsmittel um ein $C_1$-$C_6$-Alkanol handelt.

## Claims

1. A process for selectively hydrogenating citronellal to citronellol in which a liquid phase, in which the citronellal is dissolved and particles of a catalyst are suspended which is capable of preferentially hydrogenating carbon-oxygen double bonds over carbon-carbon double bonds, is conducted through a device which inhibits the transport of the catalyst particles in the presence of a hydrogen-containing gas, wherein the device inhibiting the transport of the catalyst particles has orifices or channels whose hydraulic diameter is from 2 to 2000 times the average diameter of the catalyst particles, and the liquid phase further comprises ammonia, a primary, secondary and/or tertiary amine and an inert diluent and the concentration of citronellal in the liquid phase is from 50 to 90% by weight.

2. The process according to claim 1, wherein the active component of the catalyst comprises ruthenium.

3. The process according to claim 1 or 2, wherein catalyst particles having an average diameter of from 0.0001 to 2 mm are used.

4. The process according to any of the preceding claims, wherein the device inhibiting the transport of the catalyst particles is a dumped packing, a knit, an open-celled foam structure or a structured packing element.

5. The process according to any of the preceding claims, wherein the liquid phase and the hydrogen-containing gas are conducted through the device inhibiting the transport of the catalyst particles at a superficial velocity of more than 100 $m^3/m^2h$.

6. The process according to any of the preceding claims, wherein the surfaces of the device facing toward the liquid phase have a roughness in the region of from 0.1 to 10 times the average diameter of the catalyst particles.

7. The process according to any of the preceding claims, wherein the diluent is a $C_1$-$C_6$-alkanol.

## Revendications

1. Procédé d'hydrogénation sélective de citronellal en citronellol, dans lequel on envoie une phase liquide, dans laquelle le citronellal est dissous et où se trouvent en suspension des particules d'un catalyseur apte à l'hydrogénation préférentielle de doubles liaisons carbone-oxygène plutôt que de doubles liaisons carbone-carbone, en présence d'un gaz contenant de l'hydrogène, dans un dispositif empêchant le transport des particules de catalyseur, où le dispositif empêchant le transport des particules de catalyseur présente des ouvertures ou des canaux dont le diamètre hydraulique est de 2 à 2000 fois le diamètre moyen des particules de catalyseur, et la phase liquide comprend en outre de l'ammoniac, une amine primaire, secondaire et/ou tertiaire ainsi qu'un agent de dilution inerte, et la concentration de citronellal dans la phase liquide est de 50 à 90% en poids.

2. Procédé suivant la revendication 1, dans lequel le composant actif du catalyseur contient du ruthénium.

3. Procédé suivant la revendication 1 ou 2, dans lequel on utilise des particules de catalyseur d'un diamètre moyen de 0,0001 à 2 mm.

4. Procédé suivant l'une quelconque des revendications qui précèdent, dans lequel on utilise en tant que dispositif empêchant le transport des particules de catalyseur un remplissage, un tricotage, une structure de mousse à alvéoles ouvertes ou un élément de garnissage.

5. Procédé suivant l'une quelconque des revendications qui précèdent, dans lequel on envoie la phase liquide et le gaz contenant de l'hydrogène dans le dispositif empêchant le transport des particules de catalyseur avec une vitesse en tube vide de plus de 100 $m^3/m^2h$.

6. Procédé suivant l'une quelconque des revendications qui précèdent, dans lequel les surfaces du dispositif dirigées du côté de la phase liquide présentent une rugosité de l'ordre de 0,1 à 10 fois le diamètre moyen des particules de catalyseur.

7. Procédé suivant l'une quelconque des revendications qui précèdent, dans lequel l'agent de dilution est un alcanol en $C_1$ à $C_6$.

Fig. 1

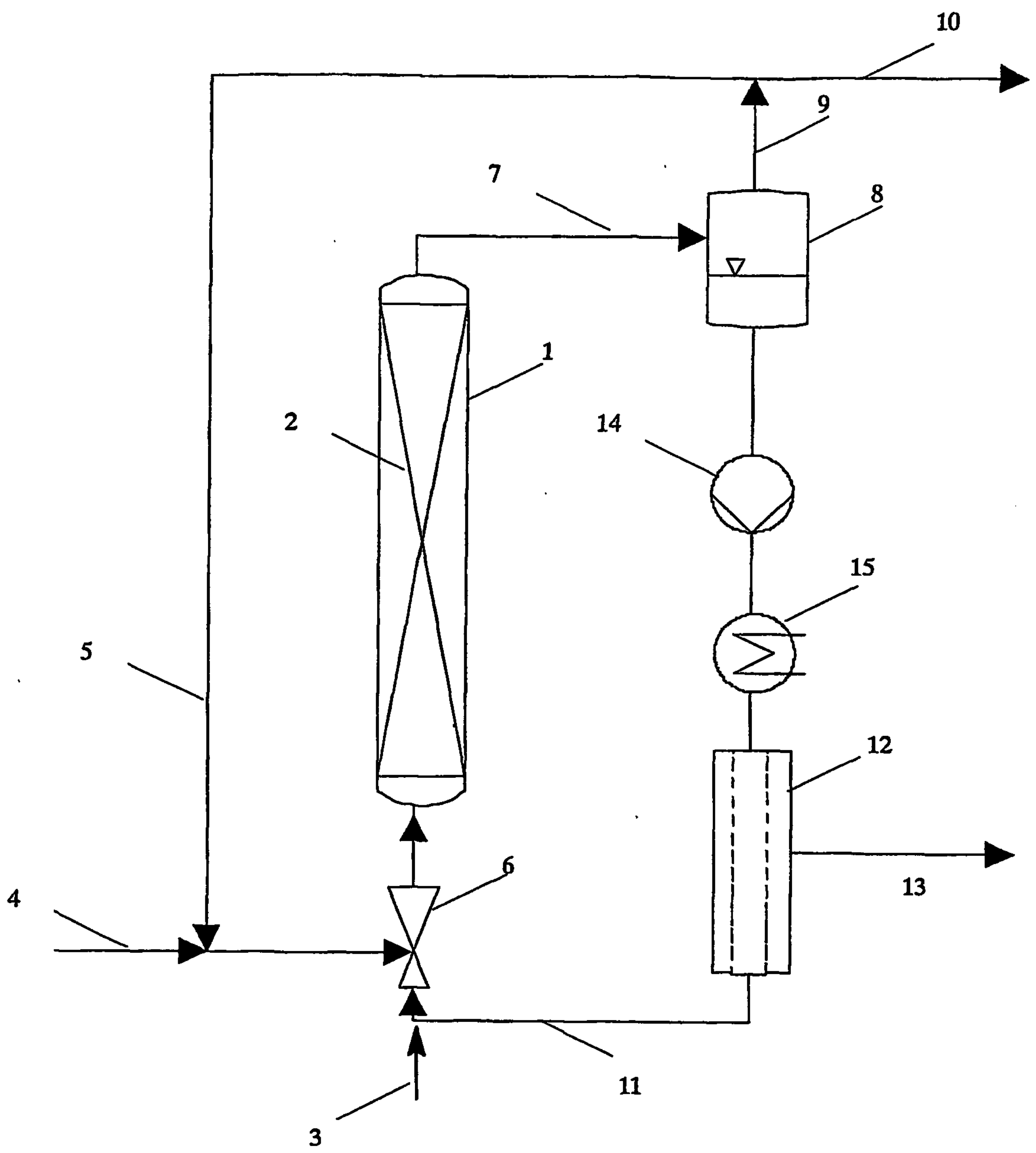

Fig. 2

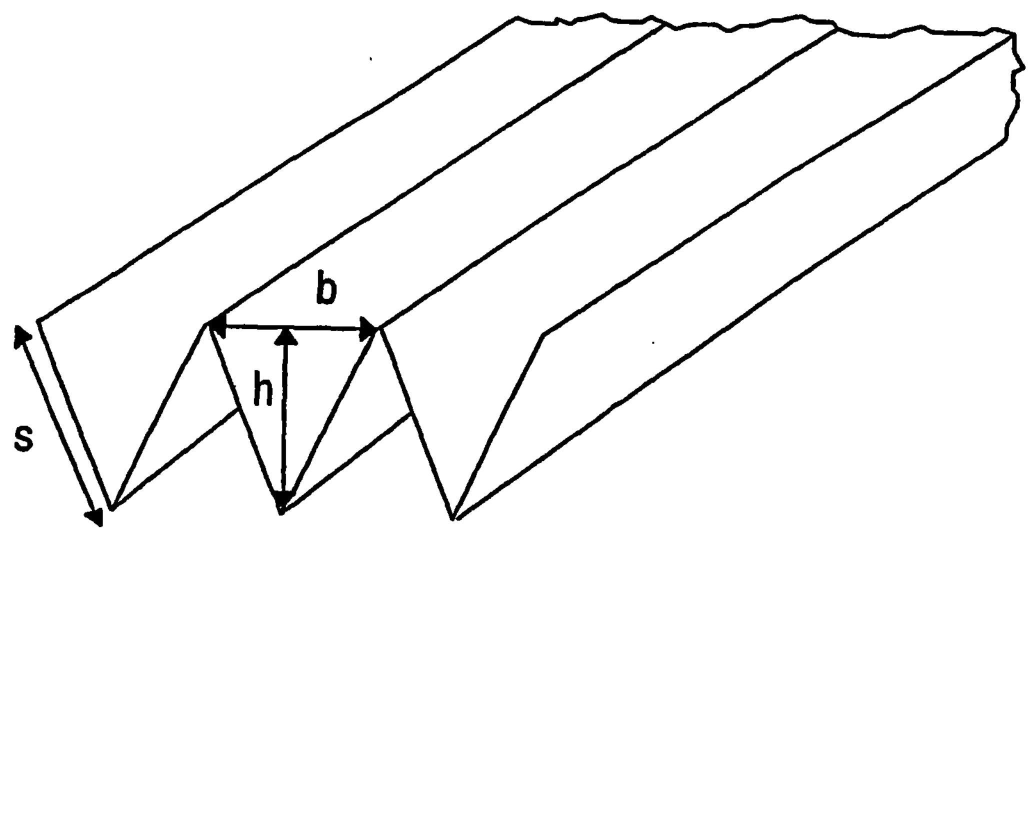

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3346650 A
- US 4029709 A
- EP 798039 A


### In der Beschreibung aufgeführte Nicht-Patentliteratur


- Ullmanns Encyklopädie der technischen Chemie. Verlag Chemie, 1997, vol. 13, 138